# FASCICULE DE BREVET EUROPEEN

(11) **EP 1 536 751 B1**
(45) Date de publication et mention de la délivrance du brevet: **08.11.2006**
(21) Numéro de dépôt: 03769610.1
(22) Date de dépôt: 10.09.2003
(51) Int. Cl.: A61K 8/34, A61K 8/97, A61Q 19/06, A61K 36/02, A61P 3/04

(54) **COMPOSITION COSMETIQUE OU PHARMACEUTIQUE COMPRENANT UN EXTRAIT D'UNE ALGUE BRUNE DU GENRE HALOPTERIS**
KOSMETISCHE ODER PHARMAZEUTISCHE ZUSAMMENSETZUNG ENTHALTEND EINEN EXTRAKT EINER BRAUNALGE DER ART HALOPTERIS
COSMETIC OR PHARMACEUTICAL COMPOSITION COMPRISING AN EXTRACT OF HALOPTERIS ALGAE

(30) Priorité: 12.09.2002 FR 0211520
(43) Date de publication de la demande: 08.06.2005
(73) Titulaire: Societe de Courtage et de Diffusion, 35417 Saint-Malo Cedex (FR)
(72) Inventeur: GEDOUIN, Antoine, F-35350 Saint-Coulomb (FR); VALLEE, Romuald, F-35350 Saint-Meloir-des-Ondes (FR); MORVAN, Pierre-Yves, F-35700 Rennes (FR)
(74) Mandataire: Maillet, Alain
(86) Numéro de dépôt international: PCT/FR2003/002691
(87) Numéro de publication internationale: WO 2004/024102

(56) Documents cités:
- WO-A-03/072118
- FR-A- 2 837 386
- R.C. PEPE, J.A. WENNINGER, G.N. MCEWEN: "International Cosmetic Ingredient Dictionary and Handbook" juin 2002 (2002-06) , THE COSMETIC, TOILETRY AND FRAGRANCE ASSOCIATION , WASHINGTON, D.C. XP002241507 021643 ISBN 1-882621-29-8 Ninth Edition, Volume I page 707, ligne 3

## Description

La présente invention concerne l'utilisation d'un extrait d'algue dans une composition cosmétique ou pharmaceutique destinée à limiter l'expansion des tissus adipeux. Elle concerne également ladite composition cosmétique ou pharmaceutique destinée à limiter l'expansion des tissus adipeux.

Les tissus adipeux sont formés d'une matrice conjonctive essentiellement constituée de cellules graisseuses du tissu hypodermique, ou adipocytes, qui sont des cellules qui renferment une volumineuse vacuole lipidique sous forme de triglycérides. L'activité métabolique de ces adipocytes comporte, d'une part, une étape de synthèse des triglycérides, que l'on appelle la lipogénèse, et d'autre part, une étape d'hydrolyse des triglycérides, que l'on appelle la lipolyse, qui va libérer dans le sang des acides gras qui sont utilisés par les autres cellules de l'organisme à des fins énergétiques.

Pour limiter une expansion des tissus adipeux, il est connu d'utiliser des substances qui peuvent agir sur les adipocytes, soit en stimulant la lipolyse des triglycérides intra-cellulaires et le re-largage extra-cellulaire des acides gras et du glycérol, soit au contraire en inhibant la lipogénèse de nouveaux triglycérides. Des extraits de plantes ayant ce type d'activité ont ainsi pu être utilisés dans la composition de produits cosmétiques et/ou pharmaceutiques destinés à affiner la silhouette.

A titre d'exemple, on peut citer le document brevet FR-A-2 693 917 qui décrit un extrait aqueux de l'algue brune *Laminaria digitata* lequel a montré *in vitro* un effet stimulant de la lipolyse des adipocytes isolés d'hypoderme humain. On peut également citer le document de brevet FR-A-2 774 905 qui décrit un extrait lipidique de graines de *Garcinia mangostana* ayant également une activité de stimulation de la lipolyse.

Toujours à titre d'exemple, on peut citer le document de brevet FR-A-2 795 958 qui décrit un extrait lipidique de la micro-algue *Odontella aurita* (riche en acides gras polyinsaturés) montrant un effet inhibiteur de la néosynthèse des acides gras dans des adipocytes isolés d'hypoderme humain et donc un effet inhibiteur de la lipogénèse ou encore le document de brevet FR-A-2 817 150 qui décrit un extrait lipidique de graines de *Polygonum fogopyrum* (riche en phytostérols) montrant un effet semblable.

La présente invention s'intéresse plus particulièrement aux algues brunes du genre *Halopteris* (ordre Sphacelaria) et, à titre d'exemple, de l'algue *Halopteris scoparia* encore nommée *Sphacelaria scoparia.* La présente invention concerne l'utilisation d'un extrait d'une algue brune du genre Halopteris pour la fabrication d'une composition pharmaceutique destinée à limiter l'expansion des tissus adipeux, ainsi que l'utilisation non-thérapeutique d'une composition cosmétique comportant au moins un extrait d'une algue brune du genre Haloperis pour limiter l'expansion des tissus adipeux. Avantageusement, cette algue est l'algue *Halopteris scoparia.*

De manière surprenante, on a pu montrer que cet extrait n'avait pas d'effets significatifs ni sur l'inhibition de la lipogénèse ni sur la stimulation de la lipolyse des triglycérides.

On a par ailleurs pu montrer, comme on le verra par la suite, que cet extrait à un effet sur la formation des adipocytes matures, seuls capables d'accumuler des triglycérides, à partir de précurseurs d'adipocytes que l'on appelle des pré-adipocytes et qui sont les seules à se multiplier. Cette transformation des pré-adipocytes en adipocytes matures est appelée différenciation cellulaire. En d'autres termes, un extrait des algues brunes du genre *Halopteris* (ou *Sphacelaria)* a un effet inhibiteur de la différenciation des pré-adipocytes en adipocytes matures.

De manière générale, la différenciation cellulaire est le plus souvent régulée aussi bien de façon positive que négative par des facteurs adipogéniques présents dans le milieu dans lequel se trouvent les cellules, tels que des hormones, des cytokines, des facteurs de croissance, des vitamines, etc. Elle est induite par une élévation ou une diminution de la concentration d'un ou de plusieurs de ces facteurs de régulation adipogénique. Ces facteurs de régulation sont produits soit par les pré-adipocytes eux-mêmes, ce qui est le cas d'une régulation dite autocrine, soit par des cellules environnantes, ce qui est le cas d'une régulation dite paracrine, soit encore par des cellules plus éloignées mais reliées aux pré-adipocytes par la voie sanguine, ce qui est le cas d'une régulation dite endocrine. Dans le cas des adipocytes, les pré-adipocytes sont des cellules de type fibroblastique qui, en fonction des facteurs environnants, peuvent se différencier soit en adipocytes dans le tissu adipeux, soit en ostéoblastes dans le tissu osseux.

On sait d'après un document paru dans *Biochem.Mol.Biol.In.,* 1994, 32, p705-p712, intitulé *"Regulation of lipoprotein lipase synthesis in 3T3-L1 adipocytes by interleukin-11*/*adipogenesis inhibitory factor"* et ayant pour auteurs Oshumi J, Miyadai K et Itoh Y que l'interleukine-11 (IL-11) présente une activité d'inhibition de la différenciation adipocytaire et constitue donc un facteur adipogénique négatif. De même, un document paru dans *J. Biol. Chem.,* 1996, 271: 615-618, intitulé *"Tumor necrosis factor promotes phosphorylation and binding of insulin receptor substrate 1 to phosphatidylinositol 3-kinase in 3T3-L1 adipocytes"* et ayant pour auteurs Guo D et Donner DB a montré un tel effet pour le facteur alpha de nécrose des tumeurs (TNFa).

Parmi les facteurs adipogèniques qui stimulent la différenciation, l'insuline et le *insulin-like growth factor* 1 (IGF-1) augmentent l'expression des marqueurs de la différenciation des adipocytes tels que les enzymes *fatty acid synthase* (FAS),. glycerol-3-phosphate dehydrogenase (G₃PDH), stearoyl CoA desaturase (SCD), ou encore des protéines membranaires comme les transporteurs de glucose (GLUT-4) et les transporteurs d'acides gras (FABP) exprimés à la surface des adipocytes humains (voir à ce sujet le document paru dans *J. Nutr.,* 1996, 126: 865-870, intitulé " Insulin increases lipogenic enzyme activity in human adipocytes in primary culture" et ayant pour auteurs Moustaid N, Jones BH et Taylor JW).

Les adipocytes matures produisent et secrètent des facteurs de secrétation considérés comme de véritables hormones spécifiques de l'adipocytes, comme notamment la leptine et l'adiponectine. Cette dernière est encore l'Acrp 30 (Adipocyte complement-related protein of 30 KDa) selon un article de Scheren and al publié en 1995 dans J.Bio.Chem, 270 26746-9 ou AdipoQ mis en évidence chez la souris selon un autre article de Hu and all, publié dans J.Bio-Chem de 1996, 271 10697-703.. Une protéine similaire a été mise en évidence dans le plasma humain et nommée GBP28 (Gelatin Binding Protein of 28 KDa) ou APM1 (Adipose Most Abundant Gene Transcript 1) d'après un article publié dans J.Biochem, 120, pages 803 à 812 de Nakano and all.

On présente ci-dessous les résultats des études qui ont été menées pour étudier l'effet inhibiteur de la différenciation adipocytaire que présentent des extraits d'algues du genre *Halopteris* et, en particulier, de l'algue *Halopteris* (ou *Sphacelaria*) *scoparia.*

Pour ce faire, on va étudier l'effet d'un extrait de l'algue *Halopteris* (ou *Sphacelaria*) *scoparia* sur une lignée de pré-adipocytes 3T3-L1 induits en adipocytes.

L'extrait de l'algue *Halopteris scoparia* est un extrait hydrosoluble qui est obtenu, dans cette étude, par macération de l'algue séchée ou lyophilisée en présence d'un solvant. Le solvant est un mélange d'eau avec un co-solvant, comme par exemple le dipropylène glycol ou le glycérol. Le rapport eau /co-solvant est compris entre 0 et 80 %, de préférence entre 30 et 50 %. On notera qu'il pourrait également n'être que de l'eau.

L'étude de l'effet de cet extrait sur une lignée de pré-adipocytes 3T3-L1 induits en adipocytes est menée selon un protocole qui est connu et qui est notamment décrit dans le document paru dans *Annu. Rev. Biochem.,* 1995, 64 : 345-373, intitulé *"Transcriptional regulation of gene expression during adipocyte differentiation"* ayant pour auteurs MacDougald OA et Lane MD.

On rappelle ci-dessous brièvement ce protocole. Les cellules pré-adipocytaires de la lignée 3T3-L1 ont été maintenues en culture en milieu de Eagle modifié par Dulbelcco (DMEM) contenant 2 mM de glutamine, 4,5 g/l de glucose, 0,11 g/l de pyruvate de sodium, 10 % de sérum de veau (SVD) et des antibiotiques (50 U pénicilline et 50 µg de streptomycine par ml de milieu). Leur différenciation en adipocytes a été provoquée de la manière suivante : dans un premier temps, les cellules pré-adipocytaires ont été cultivées à confluence pendant deux jours. Au temps que l'on appelle ci-après J0, le milieu de culture est remplacé par un milieu d'induction de la différenciation (MID) composé de DMEM contenant 10 % de sérum de veau foetal (SVF), et additionné de 0,25 mM d'isobutyl methyl xanthine (IBMX), 0,25 µM de dexamethasone et 1,74 µM d'insuline. Après deux jours d'incubation, donc au temps J2, le milieu d'induction est délicatement retiré et remplacé par un nouveau milieu de culture contenant 10 % de SVF et 0,174 µM d'insuline. Ensuite, le milieu de culture est renouvelé tous les deux jours.

Après sept jours de culture, le contenu lipidique des cellules pré-adipocytaires a été évalué par la coloration à l'huile rouge. Plus précisément, les cellules pré-adipocytaires ont été fixées au formaldéhyde à 3,7 %, puis incubées pendant 10 minutes avec une solution de colorant Red Oil (à 0,5 % dans un mélange isopropanol/eau). Après 10 minutes, le tapis cellulaire a été rincé à l'eau et les cellules pré-adipocytaires ont été lysées avec de l'isopropanol pure. La densité optique (DO) du milieu a été mesurée à 540 nm.

L'extrait de l'algue brune *Haloptéris scoparia* a été testé sur les cellules 3T3-L1 au cours de la phase d'induction par le MID qui commence donc au jour J0.

Dans des premières séries expérimentales, cet extrait a été ajouté dès le début de la différenciation (au temps J0) et maintenu présent dans le milieu jusqu'à la fixation des cellules (au temps J7), par renouvellement tous les deux jours (soit aux temps J2 et J4). L'extrait a été testé aux trois concentrations suivantes : 0,4 %, 1 % et 2,5 %. De la même manière, on a utilisé un échantillon d'acide rétinoïque à 10µM qui est connu pour inhiber la différenciation des pré-adipocytes en adipocytes, comme cela est notamment décrit dans l'article paru dans *Differentiation,* 1990, 45:119-127, intitulé "The molecular basis for inhibition of adipose conversion of 3T3-L1 cells by retinoic acid" et ayant pour auteurs Stone RL et Berlnohr DA.

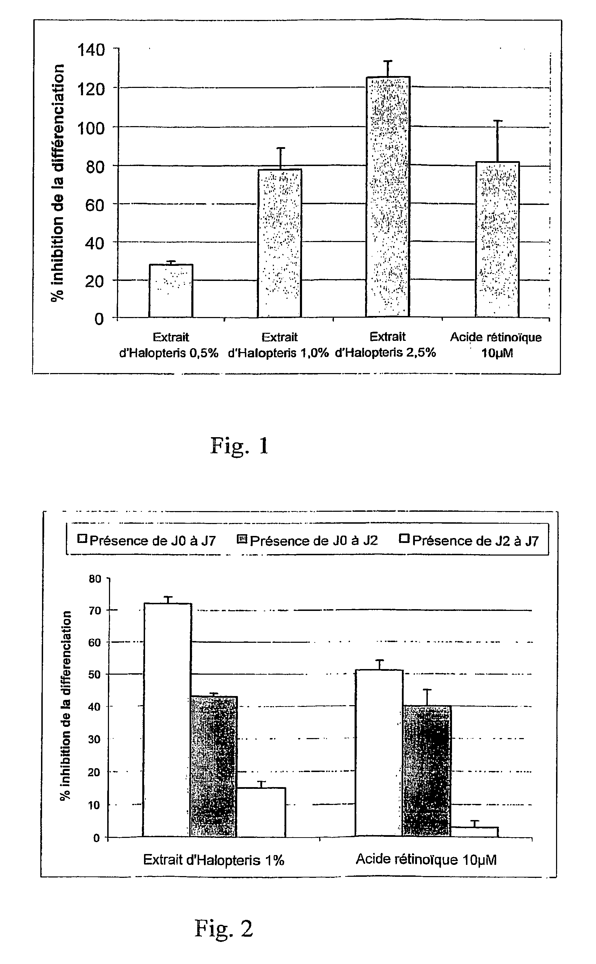

Les résultats de ces premières séries expérimentales sont présentés dans le tableau 1 ci-dessous. Des graphes correspondants sont également donnés à la Fig. 1.

**Tableau 1 : effet inhibiteur de l'extrait d'Halopteris Scopania, testé à 3 concentrations non cytotoxiques, sur la différenciation des adipocytes. Comparaison avec l'acide rétinoïque.**

| (Résultats obtenus d'une manipulation réalisée en triplicate pour l'extrait d'Halopteris, et de cinq manipulations indépendantes réalisées en triplicate pour l'acide rétinoique) | |
|---|---|
| | % inhibition de la différenciation |
| Extrait d'Halopteris 0,4 % | 28 ± 2 |
| Extrait d'Halopteris 1,0 % | 78 ± 11 |
| Extrait d'Halopteris 2,5 % | 125 ± 8 |
| Acide rétinoïque 10 µM | 82 ± 21 |

On notera que les concentrations de l'extrait *d'Halopteris* sont non-cytotoxiques. L'absence de toxicité des extraits a été évaluée sur les cellules 3T3-L1 par la méthode classique de mesure de la réduction du MTT en cristaux de formazan (voir notamment l'article paru dans *J. Immunol. Methods,* 1983, 65: 55-63, intitulé "Rapid colorimetric assay for cellular growth and survival: application to proliferation and cytotoxicity assays" et ayant pour auteur Mosmann T). Plus précisément, après incubation avec les extraits, les cellules ont été rincées et incubées pendant trois heures dans une solution de MTT préparée à 0,5 mg/ml dans un tampon phosphate (PBS). Après l'incubation, les cellules pré-adipocytaires ont été rincées en PBS et lysées dans du diméthylsulfoxide (DMSO). La DO a été mesurée à 540 nm.

Dans des secondes séries expérimentales, l'extrait a été ajouté dès le début (J0) mais, contrairement aux premières séries, il n'a pas été renouvelé par la suite, c'est-à-dire qu'il n'a pas été renouvelé après l'induction au temps J2 ni renouvelé au temps J4 et ce, jusqu'à la fixation au temps J7. Parallèlement, des cultures ont été traitées l'extrait du temps J2 au temps J7, avec renouvellement au temps J4.

Les résultats de ces secondes séries expérimentales sont présentés dans le tableau 2 ci-dessous. Des graphes correspondants sont également donnés à la Fig. 2.

**Tableau 2 : cinétique de l'effet inhibiteur de l'extrait d'Halopteris Scoparia, testé à 1%, sur la différenciation des adipocytes. Comparaison avec l'acide rétinoïque.**

| (Résultats obtenus de deux manipulations indépendantes réalisées en triplicate) | | |
|---|---|---|
| | % inhibition de la différenciation | |
| | Extrait d'Halopteris 1 % | Acide rétinoïque 10 µM |
| Présence de J0 à J7 | 72 ± 2 | 51 ± 3 |
| Présence de J0 à J2 | 43 ± 1 | 40 ± 5 |
| Présence de J2 à J7 | 15 ± 2 | 3 ± 2 |

Comme cela a été mentionné ci-dessus, dans les séries expérimentales, des cultures témoins ont été réalisées : cellules non induites (cultivées dans le milieu de culture avec 10 % SVD), et cellules induites dans le milieu MID, en absence ou en présence d'acide rétinoïque testé à 10 µM. Ce dernier est en effet connu pour inhiber la différenciation des pré-adipocytes en adipocytes.

On a également pu constater qu'en absence du milieu inducteur de différenciation (MID), les cellules restent en très forte majorité des pré-adipocytes. Seules quelques cellules produisent spontanément des gouttelettes lipidiques visibles au microscope après coloration des lipides par l'huile rouge.

De même, on a pu constater qu'en présence du milieu inducteur MID pendant deux jours, les cellules dans leur très grande majorité contiennent une multitude de gouttelettes lipidiques visibles après coloration par l'huile rouge.

Les conclusions de ces séries expérimentales sont les suivantes.

L'extrait d'*Halopteris Scoparia,* testé à 0,4 %, 1 % et 2,5 % (v/v), inhibe la différenciation des pré-adipocytes en adipocytes, de respectivement 28 %, 78 % et 125 %. Cet effet inhibiteur suit une relation dose-dépendante, comme cela est visible à la Fig. 1.

On constate qu'une inhibition supérieure à 100 % est mesurée pour l'extrait à 2,5 % : ceci montre que la présence de l'extrait à cette concentration non cytotoxique, bloque la formation de gouttelettes lipidiques qui apparaissent spontanément dans quelques cellules en absence du milieu inducteur.

On remarque que, dans les mêmes conditions expérimentales, la présence d'acide rétinoïque à la dose de 10 µM limite très fortement l'apparition des gouttelettes lipidiques : les cellules restent dans une configuration de pré-adipocytes, et ne se sont pas différenciées en adipocytes.

La présence de l'extrait d'Haloptéris, testé à 1 % (v/v), pendant seulement les deux jours d'induction par le MID (c'est-à-dire du temps J0 au temps J2) suffit à bloquer la différenciation des adipocytes : inhibition de 43 %, contre 72 % lorsque l'extrait est présent pendant toute la culture (c'est-à-dire du temps J0 au temps J7). Lorsque l'extrait est mis en contact avec les cellules après l'induction de la différenciation (c'est-à-dire du temps J2 au temps J7), il exerce un effet inhibiteur nettement plus faible : inhibition de seulement 15 % (cf. tableau 2).

Ces résultats démontrent l'importance de la présence de l'extrait dès le début de la différenciation ; un effet similaire est obtenu, dans des conditions expérimentales identiques, avec l'acide rétinoïque, testé à 10 µM (cf. Fig. 2). On notera que l'importance de la période pendant laquelle sont ajoutés les rétinoïdes pour induire une différenciation a été largement décrite par Xue JC, Schwarz EJ, Chawla A et Lazar MA dans un article paru dans *Mol. Cell. Biol.,* 1996, 16:1567-1575 sous le titre "Distinct stages in adipogenesis revealed by retinoid inhibition of differentiation after induction of PPARgamma".

De ces séries expérimentales, on peut déjà conclure qu'un extrait d'*Halopteris Scoparia* agit comme un véritable inhibiteur de la différenciation et non pas comme un inhibiteur de la synthèse lipidique (lipogénèse).

Pour corroborer les résultats qui viennent d'être décrits, on a réalisé une nouvelle série d'expériences dans laquelle l'effet d'un extrait d'*Halopteris Scoparia* sur des cellules pré-adipocytaires de la lignée 3T3-L1 a été étudié quant à l'expression des gènes codant des marqueurs habituellement exprimés par les adipocytes différenciés.

On a donc mis en oeuvre comme précédemment une phase de mise en culture à confluence des cellules pré-adipocytaires pendant deux jours, jusqu'à l'instant dit J0. On dispose alors d'un milieu qui sert de témoin. Au temps J0, le milieu de culture est remplacé par un milieu d'induction de la différenciation MID, en présence ou pas d'extrait d'*Halopteris.*

Au temps J4 et J7 (respectivement J0 + 4 jours et J0 + 7 jours), les ARN totaux ont été extraits à l'aide d'un réactif adéquat (en l'occurrence un réactif "Tri-Reagent") selon le protocole préconisé par le fournisseur. L'ADN a été éliminé (Système DNA free, Ambion). La qualité de l'ADN a été vérifié sur gel d'agarose. La réaction de Reverse Transcription (RT) de l'ARNm a été réalisée en présence de l'amorce oligo(dT) et de l'enzyme *Superscript* II (Gibco). L'ADNc synthétisé a été quantifié par fluorescence et ajusté à la concentration de 20 ng/ml. La réaction de Polymerase Chain Reaction (PCR) a été réalisée par PCR quantitative avec le système "Light cycler" de la société Roche Molecular Systems Inc. selon les procédures recommandées par le fournisseur. L'incorporation de fluorescence dans l'ADN amplifié est mesurée en continu au cours des cycles de PCR et permet d'obtenir une valeur d'expression relative pour chaque marqueur étudié.

On a ainsi étudié l'expression du facteur de transcription PPARg (Peroxisome proliferator activated receptor gamma) qui contrôlerait l'expression des gènes 422/aP2 codant pour des protéines cytoplasmiques liant les acides gras (FABP4), l'expression du facteur de transcription SREBP 1 (Sterol Response Element Binding Protein) qui contrôlerait l'expression du gène codant l'enzyme FAS (Fatty Acid Synthase), ainsi que les protéines c/EBPa, c/EBPb, c/EBPg (CAAT-enhancer-hinding protein alpha, beta et gamma). Le facteur c/EBPa contrôle l'expression des gènes de la stéaroylCoA desaturase de type 1 (SCD1) et du récepteur de glucose GLUT-4. L'expression du transporteur des acides gras (fatty acid translocase ou FAT) a également été étudié.

Les résultats obtenus sur les trois milieux (milieu témoin, milieu en présence du seul milieu inducteur et milieu en présence du milieu inducteur et de l'extrait) et, ce, pour des temps d'induction des deux seconds milieux de 4 et 7 jours sont maintenant donnés.

Après 4 jours d'induction, l'expression de c/EBPa a augmenté d'un facteur 10,4 par rapport au témoin non induit. En présence de l'extrait, elle a diminué de 33% par rapport aux adipocytes induits sans extrait. Après 7 jours d'induction, l'expression de c/EBPa a augmenté d'un facteur 27,7 par rapport au témoin non induit. En présence de l'extrait, elle a diminué de 37 % par rapport aux adipocytes induits sans l'extrait.

Après 4 jours d'induction, l'expression de SREBP 1 a augmenté d'un facteur 1,9 (+88%) par rapport au témoin non induit. En présence de l'extrait, elle a diminué de 21% par rapport aux adipocytes induits en absence de l'extrait. Après 7 jours d'induction, l'expression de SREBP1 a augmenté d'un facteur 2,7 (+166%) par rapport au témoin non induit. En présence de l'extrait, elle a diminué de 31 % par rapport aux adipocytes induits sans l'extrait.

Après 4 jours d'induction, l'expression de l'enzyme FAS n'a pas augmenté de façon significative par rapport au témoin non induit. Cependant, en présence de l'extrait, elle a diminué de 47% par rapport au témoin. Après 7 jours d'induction, l'expression de l'enzyme FAS a augmenté d'un facteur 8,3 par rapport au témoin non induit, et elle a diminué de 58% par rapport aux adipocytes induits sans extrait.

Après 4 jours d'induction, l'expression de facture de transcription SCD1 a augmenté d'un facteur de 14 par rapport au témoin non induit. En présence de l'extrait, elle a diminué de 25% par rapport aux adipocytes induits sans extrait. Après 7 jours d'induction, l'expression de SCD1 a augmenté d'un facteur 567 par rapport au témoin non induit. En présence de l'extrait, elle a alors diminué de 27% par rapport aux adipocytes induits sans extrait.

Après 4 jours d'induction, l'expression de FAT a augmenté d'un facteur 1329 par rapport au témoin non induit. En présence de l'extrait, elle a diminué l'expression de FAT de 44% par rapport aux adipocytes induits sans extrait. Après 7 jours d'induction, l'expression de FAT a augmenté d'un facteur 1528 par rapport au témoin non induit. En présence de l'extrait, cette expression a diminué de 56 % par rapport aux adipocytes sans l'extrait.

Après 4 jours d'induction, l'expression de GLUT-4 a augmenté d'un facteur 281 par rapport au témoin non induit. En présence d'extrait, elle a diminué de 37% par rapport aux adipocytes induits sans extrait. Après 7 jours d'induction, l'expression de GLUT-4 a augmenté d'un facteur 3079 par rapport au témoin non induit et elle a diminué, en présence d'extrait, de 23% par rapport aux adipocytes induits sans extrait.

Conformément aux résultats attendus, le milieu d'induction de la différenciation MID, sans présence de l'extrait d'*Halopteris,* a augmenté, par rapport au milieu de culture témoin, dans une plus ou moins grande mesure, tous les marqueurs de la différenciation des pré-adipocytes en adipocytes.

On peut constater que les résultats donnés ci-dessus montrent que l'extrait de *Sphacelaria scoparia* modifie l'expression de nombreux marqueurs de différenciation des adipocytes. Notamment, il diminue l'expression du facteur de transcription c/EBPa par rapport aux adipocytes induits sans l'extrait (de 33% après 4 jours d'induction, et de 37% après 7 jours d'induction), du facteur de transcription SREBPI (de 21% après 4 jours d'induction, et de 31% après 7 jours d'induction), de l'enzyme FAS (de 47% après 4 jours d'induction, et de 58% après 7 jours d'induction), de l'enzyme SCD1 (de 25% après 4 jours d'induction, et de 27% après 7 jours d'induction), du transporteur d'acides gras FAT (de 44% après 4 jours d'induction, et de 56% après 7 jours d'induction), du transporteur de glucose GLUT-4 (de 37% après 4 jours d'induction, et de 23% après 7 jours d'induction), de la protéine adipocytaire liant les acides gras FABP4 (de 18% après 4 jours d'induction). En revanche, il n'a pas montré d'effet significatif sur le facteur PPARg. Pourtant, l'extrait module l'expression de deux gènes qui seraient sous le contrôle du facteur PPARg : il diminue (au moins temporairement) l'expression de FABP4.

Il diminue également l'expression de la lipoprotéine lipase (LPL). Après 7 jours, d'induction l'expression de la LPL induite par le milieu d'induction est diminuée de 66 % en présence de l'extrait.

On a également pu montrer qu'après 4 jours d'induction, l'expression de COL1 a diminué de 70% par rapport au témoin non induit. En présence de l'extrait, son expression n'a diminué que de 46% par rapport au témoin non induit, ce qui signifie qu'elle a augmenté de 79% par rapport aux adipocytes induits sans extrait. Après 7 jours d'induction, l'expression de COLI a diminué de 80% par rapport au témoin non induit. En présence de l'extrait, son expression n'a diminué que de 59% par rapport au témoin non induit, ce qui signifie qu'elle a augmenté d'un facteur 2 par rapport aux adipocytes induits sans extrait.

Après 4 jours d'induction, l'expression de COL4 a augmenté d'un facteur 3,1 par rapport au témoin non induit. En présence de l'extrait, elle a augmenté 16% par rapport aux adipocytes induits sans extrait. Après 7 jours d'induction, l'expression de COL4 a augmenté d'un facteur 2,6 par rapport au témoin non induit. En présence de l'extrait, elle a augmenté de 17% par rapport aux adipocytes induits sans extrait.

Ainsi, l'addition du milieu inducteur MID a diminué l'expression du collagène de type 1 (COL1). Ce collagène est le plus abondant dans le derme. Il forme des fibres épaisses dont la principale propriété est la résistance à la traction. Il est produit principalement par les fibroblastes, mais aussi par les cellules cartilagineuses et les ostéoblastes ; ces cellules dérivent du fibroblastes comme les adipocytes. Il permet de maintenir les adipocytes attachées entre eux. Au cours de la différenciation, les pré-adipocytes perdent donc leur capacité à produire du collagène de type I. En revanche, les adipocytes matures produisent du collagène de type 4 (COL4). Celui-ci ne forme pas de fibres. Il se situe sous une forme non organisée dans les lames basales, notamment dans la membrane basale dermo-épidermique. COL4 sert de support de filtration en étant situé à l'intermédiaire entre le tissu de soutien (le derme) et le tissu épithélial (l'épiderme). II est fabriqué par les cellules épithéliales (kératinocytes) et les cellules endothéliales. Il est également produit par les adipocytes.

On peut constater que l'extrait modifie l'expression des collagènes : il diminue la perte d'expression du collagène de type 1 (COL 1) habituellement observée au cours de la différenciation. L'expression de COL 1 augmente par rapport aux adipocytes induits sans l'extrait (de 79% après 4 jours d'induction, et de 59% après 7 jours d'induction). Par ailleurs, l'extrait n'empêche pas l'expression du collagène de type 4 (COL4) qui est habituellement augmentée au cours de la différenciation des adipocytes. L'extrait augmente l'expression de COL 4 par rapport aux adipocytes induits sans l'extrait (de 16% après 4 jours d'induction, et de 17% après 7 jours d'induction).

L'analyse de l'expression des gènes de la différenciation des adipocytes par la méthode RTPCR permet donc de confirmer l'effet inhibiteur de l'extrait d'*Halopteris scoparia* sur la différenciation des préadipocytes en adipocytes matures. Elle met également en évidence que cet effet est restreint à certains marqueurs spécifiques de la différenciation. De plus, il maintient élevé le taux de collagène de type 1 (COL1), tout en stimulant l'expression du collagène de type 4 (COL4). Cet effet est intéressant compte tenu de la nécessité de restructuration du tissu dermique suite à une attaque du tissu adipeux par des agents lipolytiques.

On a également pu montrer que l'extrait *d'Halopteris Scoparia* (à 1 %) diminuait l'expression de l'adiponectine de 70 % par rapport aux adipocytes induits en absence de l'extrait.

Par conséquent, la présente invention concerne une utilisation d'un extrait d'une algue brune du genre *Halopteris* pour la fabrication d'une composition pharmaceutique destinée à limiter l'expansion des tissus adipeux, ainsi que l'utilisation non-thérapeutique d'une composition cosmétique comportant au moins un extrait d'une algue brune du genre Halopteris pour limiter l'expansion des tissus adipeux. Ladite algue est avantageusement l'algue *Halopteris scoparia.*

Par ailleurs, selon un mode de réalisation avantageux, ledit extrait est obtenu par macération d'algues séchées ou lyophilisées en présence d'un solvant, tel que de l'eau, ou un mélange d'eau avec un co-solvant. Ledit co-solvant peut être du dipropylène glycol ou le glycérol, le rapport eau /co-solvant étant compris entre 0 et 80 %, avantageusement, entre 30 et 50 %.

La présente invention concerne également une composition cosmétique ou pharmaceutique destinée à limiter l'expansion des tissus adipeux qui est caractérisée en ce qu'elle comporte en tant que produit actif au moins un extrait d'une algue brune du genre *Halopteris* qui agit de manière à inhiber la différenciation adipocytaire et en ce qu'elle comporte en outre un agent actif utile pour stimuler la lipolyse et/ou un agent actif utile pour inhiber la lipogénèse. Ladite algue est avantageusement l'algue *Halopteris scoparia ou Sphacelaria scoparia.*

Selon une caractéristique de la présente invention, on associe, dans une formulation cosmétique à visée amincissante, un extrait d'algue *d'Halopteris,* un actif stimulant la lipolyse (comme par exemple un inhibiteur de la phosphodiesterase ou un bloquant des récepteurs adrénergiques), et/ou un actif inhibiteur de la lipogénèse (comme par exemple un inhibiteur de l'enzyme *fatty acid synthase* ou un bloquant des récepteurs de pénétration du glucose). Ledit extrait est avantageusement obtenu par macération d'algues séchées ou lyophilisées en présence d'un solvant, tel que de l'eau, ou un mélange d'eau avec un co-solvant. Ledit co-solvant peut être du dipropylène glycol ou le glycérol, le rapport eau /co-solvant étant compris entre 0 et 80 %, avantageusement, entre 30 et 50 %.

La présente invention concerne également une utilisation non-thérapeutique d'une composition cosmétique telle qu'elle vient d'être décrite précédemment et ce, à des fins de limitation de l'expansion des tissus adipeux par inhibition de la différenciation pré-adipocytaire.

## Revendications

1. Utilisation d'un extrait d'une algue brune du genre *Halopteris* pour la fabrication d'une composition pharmaceutique destinée à limiter l'expansion des tissus adipeux.

2. Utilisation selon la revendication 1, **caractérisée en ce que** ladite algue est l'algue *Halopteris scoparia.*

3. Utilisation selon la revendication 1 ou 2, **caractérisée en ce que** ledit extrait est susceptible d'être obtenu par macération d'algues séchées ou lyophilisées en présence d'un solvant, tel que de l'eau, ou un mélange d'eau avec un co-solvant.

4. Utilisation selon la revendication 3, **caractérisée en ce que** ledit co-solvant est du dipropylène glycol ou le glycérol.

5. Utilisation selon la revendication 4, **caractérisée en ce que** le rapport eau /co-solvant est compris entre 0 et 80 %.

6. Utilisation selon la revendication 5, **caractérisée en ce que** le rapport eau/co-solvant est compris entre 30 et 50 %.

7. Utilisation selon l'une quelconque des revendication précédentes, **caractérisée en ce que** ladite composition pharmaceutique comporte en outre un agent actif utile pour stimuler la lipolyse et/ou un agent actif utile pour inhiber la lipogénèse.

8. Composition cosmétique ou pharmaceutique comportant au moins un principe actif extrait d'une algue brune du genre *Halopteris* destinée à limiter l'expansion des tissus adipeux, **caractérisée en ce qu'**elle comporte en outre un agent actif utile pour stimuler la lipolyse et/ou un agent actif utile pour inhiber la lipogénèse.

9. Utilisation non thérapeutique d'une composition cosmétique comportant au moins un extrait d'une algue brune du genre *Halopteris* pour limiter l'expansion des tissus adipeux.

10. Utilisation non thérapeutique d'une composition cosmétique selon la revendication 8 pour limiter l'expansion des tissus adipeux.

## Claims

1. Use of an extract of a brown alga of the genus *Halopteris* for the production of a pharmaceutical composition intended to limit the expansion of adipose tissues.

2. Use according to claim 1, **characterised in that** the said alga is the alga *Halopteris scoparia.*

3. Use according to claim 1 or 2, **characterised in that** the said extract may be obtained by maceration of dried or freeze-dried algae in the presence of a solvent, such as water, or a mixture of water and a co-solvent.

4. Use according to claim 3, **characterised in that** the said co-solvent is dipropylene glycol or glycerol.

5. Use according to claim 4, **characterised in that** the water/co-solvent ratio is between 0 and 80%.

6. Use according to claim 5, **characterised in that** the water/co-solvent ratio is between 30 and 50%.

7. Use according to any of the preceding claims, **characterised in that** the said pharmaceutical composition also includes an active agent to stimulate lipolysis and/or an active agent to inhibit lipogenesis.

8. Cosmetic or pharmaceutical composition including at least one active ingredient extracted from a brown alga of the genus *Halopteris* intended to limit the expansion of adipose tissues, **characterised in that** it also includes an active agent to stimulate lipolysis and/or an active agent to inhibit lipogenesis.

9. Non-therapeutic use of a cosmetic composition including at least one extract of a brown alga of the genus *Halopteris* to limit the expansion ofadipose tissues.

10. Non-therapeutic use of a cosmetic composition according to claim 8 to limit the expansion of adipose tissues rentiation.

## Patentansprüche

1. Verwendung eines Extrakts einer Braunalge der Gattung *Halopteris* zur Herstellung einer pharmazeutischen Zusammensetzung, die den Zweck hat, die Ausbreitung von adipösem Gewebe zu begrenzen.

2. Verwendung nach Anspruch 1, **dadurch gekennzeichnet, dass** die Alge *Halopteris scoparie* ist.

3. Verwendung nach Anspruch 1 oder 2, **dadurch gekennzeichnet, dass** der Extrakt durch Mazeration getrockneter oder gefriergetrockneter Algen in Gegenwart eines Lösemittels, wie etwa Wasser oder einer Mischung aus Wasser und einem Co-Lösemittel erhalten werden kann.

4. Verwendung nach Anspruch 3, **dadurch gekennzeichnet, dass** das Co-Lösemittel Dipropylenglycol oder Glycerol ist.

5. Verwendung nach Anspruch 4, **dadurch gekennzeichnet, dass** das Verhältnis Wasser/Co-Lösemittel zwischen 0 und 80 % beträgt.

6. Verwendung nach Anspruch 5, **dadurch gekennzeichnet, dass** das Verhältnis Wasser/Co-Lösemittel zwischen 30 und 50 % beträgt.

7. Verwendung nach einem der vorhergehenden Ansprüche, **dadurch gekennzeichnet, dass** die pharmazeutische Zusammensetzung außerdem einen nützlichen Wirkstoff umfasst, um die Lipolyse zu stimulieren und/oder einen nützlichen Wirkstoff, um die Lipogenese zu hemmen.

8. Kosmetische oder pharmazeutische Zusammensetzung, die mindestens ein Wirkbestandteil umfasst, der aus einer Braunalge der Gattung *Halopteris* extrahiert ist, die den Zweck hat, die Ausbreitung adipösen Gewebes zu begrenzen, **dadurch gekennzeichnet, dass** sie außerdem einen nützlichen Wirkstoff umfasst, um die Lipolyse zu stimulieren und/oder einen nützlichen Wirkstoff, um die Lipogenese zu hemmen.

9. Nicht therapeutische Verwendung einer kosmetischen Zusammensetzung, die mindestens einen Extrakt einer Braunalge der Gattung *Halopteris* umfasst, um die Ausbreitung adipösen Gewebes zu begrenzen.

10. Nicht therapeutische Verwendung einer kosmetischen Zusammensetzung nach Anspruch 8, um die Ausbreitung adipösen Gewebes zu begrenzen.
